# EUROPEAN PATENT APPLICATION

(11) **EP 3 293 262 A1**
(43) Date of publication of application: **14.03.2018**
(21) Application number: 16188155.2
(22) Date of filing: 09.09.2016
(51) Int. Cl.: C12N 15/10, C12N 15/65, C12N 15/66

(54) **METHOD FOR GENERATING A MULTIPLE-GENE ASSEMBLY IN A SINGLE DNA CONSTRUCT**

(71) Applicant: Universität Potsdam, 14469 Potsdam (DE)
(72) Inventor: MESSERSCHMIDT, Katrin, 14478 Potsdam (DE); MACHENS, Fabian, 16761 Henningsdorf (DE); HOCHREIN, Lena, 16761 Henningsdorf (DE); MÜLLER-RÖBER, Bernd, 14469 Potsdam (DE)
(74) Representative: Müller & Schubert

(57) **Abstract**

Described is a method for generating a multiple-gene assembly in a single DNA construct, a kit and a single DNA construct generated by the method.

Different assembly methods are known in the state of the art but user has to decide between scar free, sequence independent methods, which require the time consuming individual design of every assembly and modular approaches, which generate scar sequences and require laborious parts domestication.

In order to overcome these drawbacks a method for generating a multiple-gene assembly in a single DNA construct is provided that contains freely designed scar-free level 0 assembly units in a predefined order, determined by flanking homology regions and which is sequence-independent.

## Description

The present invention relates to a method for generating a multiple-gene assembly in a single DNA construct, a kit and a single DNA construct generated by the method.

Synthetic biology is a relatively young but fast developing scientific discipline, which combines aspects of life sciences, chemistry and engineering to design, test and build new biological systems. Many projects in synthetic biology require sophisticated cloning methods, suitable for the modular assembly of complex constructs, for example for the programmable production of compounds of medical or technical interest, construction of synthetic genomes or the design of regulatory circuits.

While cloning of single DNA parts into target vectors by using endonucleases and ligases has become a routine technique in the laboratories long ago, the assembly of multi-gene constructs, entire genetic pathways or even genomes by *de novo* assembly of DNA fragments from different sources still remains a major challenge. To enable and standardize such assembly projects, many different methods have been reported. These can be roughly separated into two groups, namely sequence-dependent and sequence-independent methods.
Knight, T. (Knight, T., 2003 Indempotent Vector Design for Standard Assembly of Biobricks, MIT Libr., http://hdl.handle.net/1721.1/21168) described one of the first assembly methods, which is the sequence-dependent BioBrick method. It is based on iterative restriction and ligation steps using a combination of four restriction endonucleases with 6-basepair (bp) recognition sites and compatible overhangs. The BioBrick principle allows only step-by-step assemblies and is therefore not suitable for large multigene constructs due to time aspects. Furthermore, recognition sites of six-cutters statistically occur every 4 kilobases (kb), making it often necessary to domesticate assembly parts by deleting undesired cut sites. This is a drawback, which many sequence-dependent methods have in common. Strategies like Golden Gate cloning, Modular Cloning System (MoClo) and Golden Braid employ two Type IIS restriction enzymes such as *Bsa*I or *Bpi*I (Engler,C. et al., 2008, A one pot, one step, precision cloning method with high throughput capability, PLoS One, 3; Weber,E. et al., 2011, A Modular Cloning System for Standardized Assembly of Multigene Constructs, PLoS One, 6, e16765 and Sarrion-Perdigones, A. et al., 2011, GoldenBraid: An iterative cloning system for standardized assembly of reusable genetic modules, PLoS One, 6). The reduced number of enzymes minimizes the number of 'forbidden' DNA-sequences. Type IIS restriction enzymes cut the DNA sequence several bases away from their recognition site. In this way, a single enzyme can generate different overhangs, allowing the directed assembly of multiple DNA fragments in one step. Nevertheless, the definition of the parts position requires the time consuming subcloning of DNA parts into special vectors possessing appropriate adapter sequences and the deletion of restriction enzymes in given DNA sequences. In addition, these methods are non-reversible because the restriction sites are lost through the assembly process.
To overcome the drawbacks of restriction enzyme-based cloning methods, sequence-independent cloning methods have been developed. Such cloning methods avoid restriction enzymes and are often based on short overlapping sequences between the assembly parts, thereby allowing the directed assembly of multiple fragments into a single construct. These methods include the *in vitro* Gibson assembly (Gibson, D.G., 2011, Enzymatic Assembly of Overlapping DNA Fragments, in Christopher, V. (ed), Methods in enzymology, Academic Press, Vol. 498, pp. 349-361), the bacterial cell extract-based DNA cloning technique SLiCE described by Zhang, Y. *et al.* (Zhang,Y., Werling, U. and Edelmann W., SLICE: a novel bacterial cell extract-based DNA cloning method, Nucleic Acids Res., 40, e55) and the *in vivo* transformation-associated recombination (TAR) in *Saccharomyces cerevisiae* (Gibson,D.G., 2009, Synthesis of DNA fragments in yeast by one-step assembly of overlapping oligonucleotides, Nucleic Acids Res., 37, 6984-6990; Kouprina,N. and Larionov,V., 2008, Selective isolation of genomic loci from complex genomes by transformation-associated recombination cloning in the yeast Saccharomyces cerevisiae, Nat. Protoc., 3, 371-377). The necessary overlaps between the assembled parts are usually added via PCR amplification. While this allows sequence-independent cloning without restriction enzymes, the PCR amplification steps introduce the risk of PCR-born sequence errors. Additionally, the overlapping regions for each assembly reaction have to be designed individually depending on the sequence of neighbouring assembly parts. This results in a lack of modularity and an increased design and planning effort. Nevertheless, these methods are widely used, because they allow the easy, fast and scar-free assembly of transcriptional units and smaller constructs. However, they are not ideally suited for large multi-gene assemblies due to limitations in fragment number and requirement for individual designed overlaps.
An example for a modular, but still sequence-independent assembly strategy is MODAL, described by Casini, A. *et al.* (Casini,A., MacDonald,J.T., Jonghe,J. De, Christodoulou,G., Freemont,P.S., Baldwin,G.S. and Ellis,T., 2014, One-pot DNA construction for synthetic biology: the Modular Overlap-Directed Assembly with Linkers (MODAL) strategy, Nucleic Acids Res., 42, e7). MODAL especially focuses on high modularity for easy shuffling of single parts. Universal prefix and suffix adapters for upstream and downstream primer binding are fused to individual assembly parts, which are then kept in storage plasmids. Specific linker sequences, computationally optimized for the overlap-guided assembly with neighbouring fragments are then fused to these adapters via a PCR step. While MODAL allows the assembly of parts in different orders and orientations, the user has to accept large scar sequences, the risk of internal recombination, because every part contains the same adapter sequence and due to the necessary amplification steps, the risk of PCR-born errors with every new assembly reaction. Based on MODAL, the further developed method BASIC by Storch, M. *et al.* (Storch, M., Casini, A., Mackrow, B., Fleming, T., Trewhitt, H., Ellis, T. and Baldwin, G.S., 2015, BASIC: A New Biopart Assembly Standard for Idempotent Cloning Provides Accurate, Single-Tier DNA Assembly for Synthetic Biology, ACS Synth. Biol., 10, 1021/sb500356d) overcomes the disadvantage of PCR steps but gets back to the use of restriction enzymes and introduces even larger scar sequences. Domesticated assembly parts are released from storage plasmids, using Type IIS restriction enzymes and are than fused to linker oligonucleotides, which allow directed assembly via compatible, single stranded overhangs. While MODAL and BASIC, due to the long scar sequences are methods only applicable for the assembly of premade transcription units, the yeast Golden Gate method has been described by Agmon, N. *et al.* (Agmon,N., Mitchell,L.A., Cai,Y., Ikushima,S., Chuang,J., Zheng,A., Choi,W.-J., Martin,J.A., Caravelli,K., Stracquadanio,G. and Boeke, J.D., (2015) Yeast Golden Gate (yGG) for the Efficient Assembly of S. cerevisiae Transcription Units, ACS Synth. Biol., 4, 853-859) and is specialized on the *de novo* assembly of transcription units using Type IIS restriction enzymes but only in a predefined assembly syntax. Vegas, a method for pathway assembly in S. *cerevisiae* described by Mitchell, L.A. *et al.* (Mitchell, L.A., Chuang, J., Agmon, N., Khunsriraksakul, C., Phillips, N.A., Cai, Y., Truong, D.M., Veerakumar, A., Wang, Y., Mayorga, M. et al., 2015, Versatile genetic assembly system (VEGAS) to assemble pathways for expression in S. cerevisiae, Nucleic Acids Res., 43, 6620-6630) uses transcription units already assembled by yGG and adds so called 'Vegas adapters' to direct the overlap-based assembly into multi-gene constructs. Flexibility, with respect to the parts order, is gained by adding new overlaps to yGG-assembled transcription units via PCR amplification. This could be a problem if large parts are included and imposes the risk of PCR-born errors.

The different assembly methods described here are all efficient and suitable for many research projects. However, in most cases the user has to decide between scar free, sequence independent methods, which require the time consuming individual design of every assembly and modular approaches, which generate scar sequences and require laborious parts domestication.

It is an object of the invention to overcome the drawbacks of the single assembly methods described in the state of the art as well as to provide a new method for generating a multiple-gene assembly in a single DNA construct that contains freely designed scar-free level 0 assembly units in a predefined order, determined by flanking homology regions and which is sequence-independent.

Another object of the invention is to provide a kit in order to perform the method for generating a multiple-gene assembly in a single DNA construct.

A further object of the invention is to provide a single DNA construct which is generated by a method for assembling multiple-genes in one single DNA construct.

The problem is solved, according to the present invention, by a method for generating a multiple-gene assembly in a single DNA construct, which is characterized by the following steps:
a) Predetermining the order and orientation of subunits, which are provided as single- or double-stranded DNA, in the multiple-gene assembly in the single DNA construct to be generated,
b) Providing at least one Level 0 vector set containing at least two Level 0 vectors, which differ in their homology regions, at least one subunit per Level 0 vector and at least one Level 1 vector;
c) Providing at least one Level 2 DNA vector if at least two Level 1 vectors are provided in step b),
d) Adding overlaps to subunits in order to achieve the predetermined order and orientation of subunits,
e) Assembling the subunits of step d) into individual Level 0 vectors, depending on the predetermination in step a), chosen from one Level 0 vector set, by releasing a cassette, which separates the homology regions in each Level 0 vector, and cloning the predetermined subunit in-between the homology regions of each Level 0 vector by overlap based cloning,
f) Repeating steps d) and e) depending on the number of subunits provided in step b), whereas those steps are performed in parallel,
g) Releasing all Level 0 assembly units, which are the assembled subunits of all steps e) combined with the homology regions of the Level 0 vector, from each Level 0 vector backbone and isolating the released Level 0 assembly units,
h) Assembling the released Level 0 assembly units of step g) into one corresponding Level 1 vector by overlap based cloning between the released Level 0 assembly units and the corresponding Level 1 vector,
i) Repeating steps d) to h) in parallel for each Level 0 vector set provided in step b),
j) Releasing all Level 1 modules, which are the subcloned Level 0 assembly units combined with the homology regions of the corresponding Level 1 vector generated in steps h) and i) from the Level 1 vector backbone and isolating the released Level 1 modules,
k) Assembling of all released Level 1 modules of step j) into one single Level 2 DNA vector by overlap based cloning between the Level 1 modules and the Level 2 vector by recombination.

The overlaps in step d) are added according to the present invention by PCR-amplification or any other suitable method known in the art.

The cassette in step e) is according to the present invention an expression cassette. Especially preferred is the cassette to be a *ccdB* cassette. Advantageously in using a *ccdB* cassette is, that during the Level 0 cloning process, the *ccdB* gene reduces the empty vector background and obviates purification of the linearized Level 0 vector backbone.

Regarding step e) the cassette is either flanked by two identical restriction enzyme recognition sites or by two different restriction enzyme recognition sites. Using two different restriction enzyme recognition sites has the advantageous effect that a vector religation during the Level 0 assembly reaction is avoided.

Further, according to the present invention, the release performed in steps g) and j) is done by means of digestion or PCR amplification.

In case of the digestion according to steps g) and j) either rare cutting restriction enzymes or homing endonucleases are used. In case of restriction enzymes, eight-cutter restriction enzymes, which are *Asc*I, *Sbt*I, Swal, *Fse*I or *Pme*I, are preferably used. In respect of the homing endonuclease either I-Scel or I-Ceu*I*, or a combination of them is preferably used. The extremely rare and asymmetric 18 bp recognition site of I-SceI as well as the 27 bp recognition site of I-*Ceu*I, minimizes the chance of unintended cut sites occurring inside the Level 1 module, making it effectively unnecessary to modify any parts before the assembly. In a further realisation it is possible to use any other suitable nuclease known in the art.

Isolation by means of digestion makes it unnecessary to PCR amplify the Level 0 assembly units or the Level 1 modules, prior to subsequent Level 1 or Level 2 module assemblies, respectively, and eliminates the risk of PCR-born errors. As rare cutting restriction enzymes and homing endonucleases with cutting sites that occur rarely are used, it is highly unlikely that a subunit cloned into a Level 0 vector backbone contains the cutting sites of the rare cutting restriction enzymes or the homing endonuclease sites and thus the Level 0 assembly unit could not be released by digestion. This makes it virtually unnecessary to perform site directed mutagenesis or other laborious modifications to any part of the Level 0 assembly unit prior to cloning.

Regarding step k), the recombination is either performed *in vitro* or *in vivo.* According to the present invention it is especially preferred that the *in vivo* recombination is performed in *Saccharomyces cerevisiae.*

Preferred is the method, according to the present invention, wherein a set of optimized 15-80 bp long overlap regions, whereby 15-36 bp long overlap regions are preferred, are added to specific subunits prior to step d), thereby allowing either predetermined or combinatorial assemblies with different subunits being randomly assembled at one or more positions of a Level 0 assembly unit.

Especially preferred is the method according to the present invention wherein the vector backbone is identical for all Level 0 vectors in step e), whereas the homology regions vary in between the Level 0 vectors of one Level 0 vector set. In another alternative the Level 0 vectors of one Level 0 vector set differ not only in their homology regions but also in their vector backbones.

Further preferred is the method according to the present invention, wherein the Level 0 vectors are positioning vectors, defined by their homology regions, whereby the choice of a Level 0 vector determines the position of the corresponding Level 0 assembly unit in the multiple-gene assembly to be generated.

Especially preferred is the method according to the present invention, wherein each Level 0 vector set contains one Level 0 vector with a first homology region, which is 15 bp to 80 bp long, whereby a length of 50 bp is preferred, and corresponds to the left arm of a specific corresponding Level 1 vector and a second homology region, which is 15 bp to 80 bp long, whereby a length of 36 bp is preferred, and corresponds to the next Level 0 vector within the Level 0 vector set, whereas the homology regions of the other Level 0 vectors of one Level 0 vector set have inlying homology regions, which are both 15 bp to 80 bp long overlaps, whereby a length of 36 bp is preferred, and are required for the directed assembly of the Level 0 assembly units into single Level 1 vectors and whereas each Level 0 vector set contains one Level 0 vector with a last homology region, which is a group of 15 bp to 80 bp sequences, whereby 51 bp sequences are preferred, and correspond to the right arm of the corresponding Level 1 vector, whereby this group of sequences each represents the first codons from different selectable or screenable marker genes, is preceded by the corresponding promoter region and followed by a stop codon.

According to the present invention the selectable or screenable marker genes are auxotrophic or antibiotic resistance marker genes.

Advantageously, the first homology region, which is preferably 50 bp long that overlaps to the Level 1 vector, supports the TAR mediated assembly of large fragments in the final Level 2 assembly step. The second homology regions, which are preferably shorter and of a length of 36 bp, are sufficient for the assembly of Level 1 modules from Level 0 assembly units. Further, the stand-alone promoter region, which precedes the first codons of different selectable or screenable marker genes, fulfils two functions: it provides a terminal right arm overlap to a specific Level 1 vector and allows the expression of a selectable or screenable marker, once the next assembly level is reached. Therefore, this homology region must be present in every assembly step from Level 0 to Level 1.

Alternatively the homology regions are optimized in such a way that they are used for shuffling promoters, coding sequence (CDS) or terminators. Exemplary an optimized homology region contains yeast Kozak sequence within a 5'UTR, which results in the effect that such a homology region can be inserted in front of any CDS, whereas a homology region which contains a universal 3'UTR can be inserted behind any CDS.

Preferred is also the method according to the present invention, wherein in step e) a Level 0 vector is used, which is universal, whereby the vector is defined by two non-variable homology regions, in-between those regions any subunits are assembled and the complete Level 0 assembly unit is afterwards released and assembled into a Level 1 vector at any desired position. This is done by using for example the Paper-Clip method or any other method that allows directed assembly of two or more DNA fragments that do not possess overlapping ends.

Especially preferred is also the method according to the present invention, whereby in step h) the Level 1 vector contains one homology region, which is a 15 bp to 80 bp long region, whereby a 50 bp long region is preferred and a second homology region that is a group of 15 bp to 80 bp sequences, whereby 51 bp sequences are preferred, which correspond to the first codons of a selectable or screenable marker gene, in order to be compatible with the different Level 0 assembly units.

According to the present invention is the method preferred, wherein in step k) the Level 2 vector contains a left homology region, which corresponds to the first homology region of the first Level 1 module and a second homology region, which corresponds to the last homology region of the last Level 1 module which is part of the gene assembly.

Further, preferred is the method according to the present invention wherein the marker gene is different between the Level 0 vector and Level 1 vector and/or between the Level 1 vector and the Level 2 vector.

The marker gene according to the present invention may be any screenable or selectable marker gene known by a person skilled in the art which are for example antibiotic resistance marker genes or auxotrophic markers. This may also include dominant resistance markers such as G418.The use of different selections or screenings between the different assembly Levels has the advantageous effect that an efficient counter selection of an undigested vector backbones is possible.

Especially preferred is the method according to the present invention in that in a further step I) the Level 2 module, which are the combined Level 1 modules and homology regions of the Level 2 vector, is released from the Level 2 vector backbone and subcloned into varying vectors by either ligation based cloning or by overlap based cloning using the homology regions flanking the Level 2 module.

Further, especially preferred is the method according to the present invention in which any unit or module in Level 1 or Level 2 vector is genome integrated by using a suitable nuclease along with donor DNA, if required. The suitable nuclease is preferred to be a Cas9 protein directed by a sgRNA.

Furthermore it is also possible to integrate a cassette, such as used in the Level 0 vector, into the Level 1 and Level 2 vectors in order to enable gene selection in *Escherichia coli.*

The problem of the invention is further solved by a single DNA construct generated by the method according to the present invention, which is characterized in that it contains freely designed scar-free Level 0 assembly units in a predefined order, determined by flanking homology regions and is sequence-independent.

Further the problem of the present invention is solved by a kit for generating a single DNA construct by using the method according to the present invention comprising at least one Level 0 vector set, containing at least two Level 0 vectors, which differ in their homology regions, at least one subunit per Level 0 vector, at least one Level 1 vector and a Level 2 vector.

The problem of the invention is also solved by a single DNA construct obtainable by
a) Predetermining the order and orientation of subunits, which are provided as single- or double-stranded DNA, in the multiple-gene assembly in the single DNA construct to be generated,
b) Providing at least one Level 0 vector set containing at least two Level 0 vectors, which differ in their homology regions, at least one subunit per Level 0 vector and at least one Level 1 vector;
c) Providing at least one Level 2 DNA vector if at least two Level 1 vectors are provided in step b),
d) Adding overlaps to subunits in order to achieve the predetermined order and orientation of subunits,
e) Assembling the subunits of step d) into individual Level 0 vectors, depending on the predetermination in step a), chosen from one Level 0 vector set, by releasing a cassette, which separates the homology regions in each Level 0 vector, and cloning the predetermined subunit in-between the homology regions of each Level 0 vector by overlap based cloning,
f) Repeating steps d) and e) depending on the number of subunits provided in step b), whereas those steps are performed in parallel,
g) Releasing all Level 0 assembly units, which are the assembled subunits of all steps e) combined with the homology regions of the Level 0 vector, from each Level 0 vector backbone and isolating the released Level 0 assembly units,
h) Assembling the released Level 0 assembly units of step g) into one corresponding Level 1 vector by overlap based cloning between the released Level 0 assembly units and the corresponding Level 1 vector,
i) Repeating steps d) to h) in parallel for each Level 0 vector set provided in step b),
j) Releasing all Level 1 modules, which are the subcloned Level 0 assembly units combined with the homology regions of the corresponding Level 1 vector generated in steps h) and i) from the Level 1 vector backbone and isolating the released Level 1 modules,
k) Assembling of all released Level 1 modules of step j) into one single Level 2 DNA vector by overlap based cloning between the Level 1 modules and the Level 2 vector by recombination.

The present invention is explained in detail by the attached figures, whereas
Fig. 1 shows the workflow for the assembly of 25 parts as a first example of the present invention;
Fig. 2 shows exemplary the design of Level 0 vectors;
Fig. 3 shows exemplary a Level 0 assembly with overlap based cloning methods;
Fig. 4 shows exemplary a Level 1 assembly; and
Fig. 5 shows exemplary a Level 2 assembly.

The inventors developed a new DNA assembly strategy, whereby the most important requirements they intended to fulfil were: scar-less and sequence-independent de novo assemblies of assembly parts on gene level, standardized assemblies into multi-gene constructs, a minimum of PCR amplification steps, and a user-oriented workflow that allows even inexperienced inventors to start their assemblies right away. Furthermore, the invention is designed for use with cost efficient cloning methods and in a way that allows the user to freely choose the final host organism for gene expression.
To achieve the fast assembly of multiple parts, the strategy is based on different levels: Level 0 for the scar-free assembly of individual subunits, Level 1 for the assembly of Level 0 assembly units into one vector, and Level 2 for the assembly of Level 1 modules into a single DNA construct.

### Level 0 assembly

General design of the Level 0 entry vector, whereby the backbone is identical for all Level 0 vectors (vectors with different backbones are also possible) and differs only in the homology regions. Those homology regions are variable (a vector with two non-variable regions is also possible) and are separated by a cassette flanked by restriction sites. The restriction sites are used to release the cassette and to allow the subsequent cloning of an individual assembly unit (composed of multiple subunits) in-between the homology regions by scar-free, overlap-based cloning. Alternatively, the Level 0 vector backbone is PCR amplified to generate the linear backbone necessary for the assembly reaction. For the Level 0 assembly reaction, all subunits are equipped with customized overlaps to neighbouring subunits or to the vector sequence, respectively. This is usually achieved by introducing the overlaps with primers during PCR amplification. All PCR products, necessary for the assembly, are than combined with the linearized (or PCR amplified) Level 0 vector backbone in an appropriate assembly reaction. A successfully assembled Level 0 assembly unit is released from the backbone by using rare restriction enzyme recognition site or homing endonuclease recognition site present upstream and downstream of the cloning site.

Level 0 vectors allow the cloning of individual subunits and serve as positioning vectors for the ultimate multi-gene assembly. The choice of the Level 0 vector for cloning of a given Level 0 assembly unit defines its position in the final assembly, based on predefined homology regions present in individual Level 0 vectors: the upstream (left) homology region defines the upstream neighbour, whereas the downstream (right) homology region defines the downstream neighbour. To realize this, each downstream homology region is used as upstream region in the neighbouring downstream Level 0 vector, and so on.

Level 0 vectors are grouped into vector sets. All Level 0 assembly units of one Level 0 vector set, that have been assembled from subunits cloned in one set of Level 0 vectors, are subsequently assembled into one specific Level 1 vector. The resulting Level 1 module holds the Level 0 assembly units from one Level 0 vector set and its position within the final construct is defined by the Level 1 vector.

### Level 1 assembly

Level 1 vectors allow the assembly of the Level 0 assembly units of one Level 0 vector set into one Level 1 module. This is achieved by digestion of the Level 0 assembly units with a provided restriction enzyme or a homing endonuclease, clean-up of the DNA fragments and further combination of Level 0 assembly units with appropriate linearized Level 1 vector in an *in vitro* or *in vivo* assembly reaction. Alternatively PCR amplification is performed to achieve Level 0 assembly units. The Level 1 vectors differ in their marker gene for selection and screening compared to the Level 0 vectors. The Level 1 vectors are compatible with the different Level 0 vector sets due to their homology regions, whereby two homology regions provide overlaps to the outmost homology regions from one Level 0 vector set and the third homology region is used for later Level 2 module assembly.
The Level 1 assembly is done by *in vitro* methods or by *in vivo* assembly. The *in vivo* assembly is preferably performed in yeast. The highly efficient, but more time consuming assembly in yeast is assisted by the use of promoterless markers on the Level 1 vector backbones, which become functional only upon recombination with the appropriate Level 0 assembly unit, thereby indicating a successful assembly.

Level 1 modules are maintained in S. *cerevisiae* and/or *E.coli* and can be used directly for expression.

Homing endonucleases such as I-SceI or I-*Ceu*I or rare cutting restriction enzymes are used to release successfully assembled Level 1 modules from the Level 1 vector backbone, without the need for PCR amplification. Alternatively PCR amplification of the Level 1 assembled Level 1 modules may also be used.

### Level 2 assembly

Different Level 1 modules, each assembled from Level 0 assembly units, are combined into a single Level 2 destination vector. These vectors can be used directly to maintain and express the assembled units in *S*. *cerevisiae,* either in high or low copy number, or in *E. coli.*
The basic Level 2 vector backbones are identical to the Level 1 backbones and differ only in their homology regions. The different Level 2 vectors are designed to hold different numbers of Level 1 modules. A Level 2 vector for only one Level 1 module is not necessary, because it would be identical to the corresponding Level 1 construct. All Level 2 vectors contain a homology region providing overlap to the first Level 0 assembly unit of the first Level 1 module that is part of the assembly and to the outmost homology region from the last Level 1 module that is part of the assembly. For the final assembly step, Level 1 modules are released from the Level 1 vector backbone by restriction with either a rare cutting restriction enzyme or a homing endonuclease. Alternatively PCR amplification may also be used. The released Level 1 modules contain all necessary homologies to each other and to the linearized Level 2 vector to allow a directed final assembly.

If the final construct is intended for use in host organisms different from S. *cerevisiae* or *E. coli,* subcloning in any desired expression vector is possible with minimal effort. For this, the assembly product needs to be isolated and digested with a homing endonuclease corresponding to the Level 2 vector to release the Level 2 module. The desired vector is made compatible simply by either adding a cassette flanked by homing endonuclease recognition sites for ligation based subcloning, or a homology region cassette containing the two outmost homology regions and a suitable restriction site for overlap based cloning.

Furthermore, the inventors developed a web-based planning tool, which ensures that users with no experience in multi-part assemblies will have easy access to the concepts of the invented method and kit and minimizes the time required to design and plan the assembly.

The following example explains the invention in more details. It is not intended that the example restricts the scope of the invention.

To show the efficiency of the invention, the inventors have successfully performed a de-novo assembly of a 25 gene construct, over 72 kb in length, from 69 subunits in about four weeks.

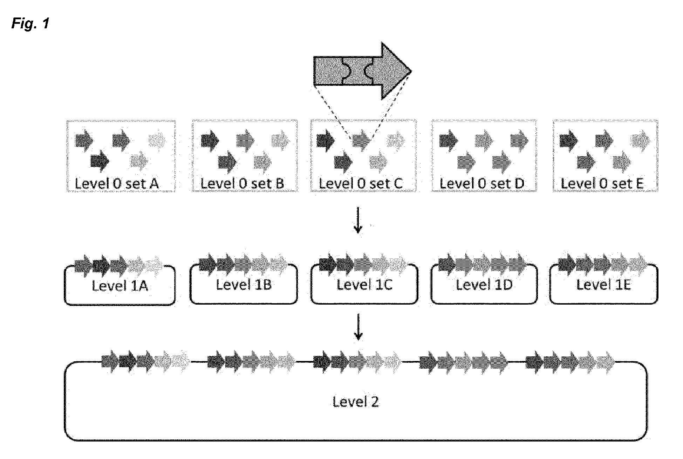

The assembly strategy presented here allows the assembly of up to 25 (transcriptional) subunits into a single construct in a predefined order. To facilitate a fast assembly process, it is designed in three cloning Levels as shown in Fig. 1: The entry Level 0 for assembly of subunits from individual parts like promoters, terminators and CDS into Level 0 assembly units, Level 1 for the assembly of multiple Level 0 assembly units into Level 1 modules, and Level 2 for the final assembly of Level 1 modules into a single construct.

As shown in Fig. 1, all Level 0 subunits (big arrow with three-parts) are assembled into five different sets of Level 0 vectors (Level 0 set A, B, C, D and E) resulting in 25 Level 0 subunits (small arrows with different shades of grey). In the next step Level 0 subunits from one Level 0 set are assembled into one corresponding Level 1 module (Level 1A, 1B, 1C, 1D and 1E). The five Level 1 modules each containing 5 Level 0 assembly units are then assembled into one single Level 2 vector.

### General design of the Level 0 entry vector

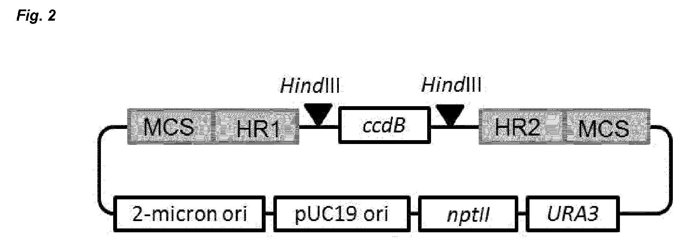

Fig. 2 shows a yeast shuttle vector as vector backbone, which is identical for all Level 0 vectors. The backbone consists of an *E. coli* replication origin *(pUC19* ori), a kanamycin resistance gene (*npt*II), a 2-micron origin for replication in yeast (2-micron ori) and a marker gene (*URA3*) for selection in S. *cerevisiae.* Each Level 0 vector possesses a different combination of homology regions (HR1, HR2). HR1 gives homology to the Level 1 vector backbone or to the HR2 of the adjacent Level 0 vector. HR2 gives homology to the next Level 0 vector or to the Level 1 vector backbone. These regions are separated by a *Hind*III-flanked *ccdB* expression cassette. The *Hind*III sites are used to release the *ccdB* cassette and to allow the subsequent cloning of the individual assembly unit (composed of multiple subunits) in-between the homology regions by scar-free, overlap-based cloning. The homology regions are flanked by two multiple cloning sites (MCS), containing the five 8-base cutters *Asc*I, *Sbt*I*, Swa*I, *Fse*I, *Pme*I to release Level 0 constructs for further cloning into Level 1 vectors.

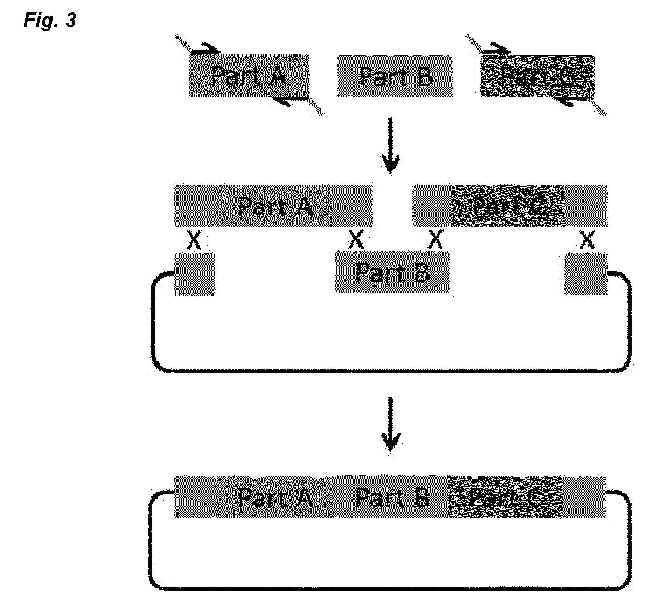

For the Level 0 assembly with overlap based cloning methods, the subunits are equipped with customized overlaps to neighbouring subunits or to the vector sequence, respectively. This is usually achieved by introducing the overlaps (marked as small x) with primers during PCR amplification, as shown in Fig. 3. After amplification of part A and part C with appropriate primers (arrows on the top and the bottom of part A and part C), those parts are assembled together with part B into a vector backbone as shown in Fig. 3. Appropriate assembly reactions for cloning the PCR amplified parts into the vector backbone are for example SLICE or Gibson assembly for overlap based cloning.

During the Level 0 cloning process, the *ccdB* gene reduces the empty vector background and obviates purification of the linearized Level 0 backbone.

### Design of Level 0 vector sets

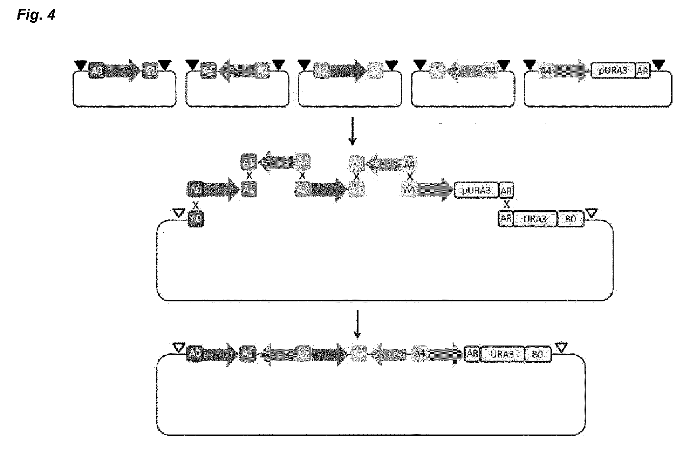

The Level 0 vectors are grouped into five sets, each containing five positioning Level 0 vectors. Set A, for example, contains vectors with homology regions A0-A1, A1-A2, A2-A3, A3-A4 and A4-AR. This set defines the position of the first five units in the final assembly product. All units, cloned in one set of Level 0 vectors, are subsequently assembled into one specific Level 1 vector as shown exemplary for set A in Fig. 4. The resulting Level 1 module holds up to five Level 0 assembly units and its position within the final construct is defined by the Level 1 vector.

As shown in Fig. 4 five Level 0 assembly units are released from Level 0 vector set A by digestion with one of the designated eight-cutters. During *in-vivo* or *in-vitro* assembly, overlaps (marked as small x) between the different Level 0 assembly units and the Level 1 destination vector ensure a directed assembly into a single circular DNA construct. In front of the homology region AR is the *URA3* promoter (p*URA3*) located. AR itself is the first 51 nucleotides of the *URA3* gene. The whole *URA3* gene and the appropriate terminator are located on a Level 1 vector backbone as shown in Fig. 4. Thus, a functional selection marker after successful Level 1 assembly is achieved.

The first (or left) homology region of a Level 0 vector set (A0, B0, C0, D0, E0) is always 50 bp long and provides the left arm homology to a specific Level 1 vector. To allow cloning into the corresponding Level 1 vector, this region must be present in every assembly of a Level 1 module from multiple Level 0 units. The inlying homology regions, e.g. A1 and A2, are 36 bp long each and provide the necessary overlaps for a directed assembly of Level 0 assembly units into a single Level 1 module. The longer overlap to the Level 1 vector supports the TAR mediated assembly of large fragments in the final Level 2 assembly step. The shorter 36 bp homology regions are sufficient for the assembly of Level 1 modules from Level 0 assembly units. Additionally, a group of 51 bp sequences, providing homology to the different Level 1 destination vectors were defined manually. Each represents the first 17 codons from one out of five different auxotrophic yeast marker genes, preceded by the corresponding promoter region and followed by a stop codon. This stand-alone promoter region fulfils two functions: it provides a terminal right arm overlap to a specific Level 1 vector and allows the expression of an auxotrophic marker, once the next assembly level is reached.

To allow the assembly of less than five units, Level 0 vectors have been developed that contain the last homology region of a vector set, providing homology to the Level 1 vector, in combination with appropriate homology regions to allow cloning of less than five genes, e.g. A0 AR and A1 AR for cloning and subsequent Level 1 assembly of only one or two units in vector set A.

### Design of Level 1 vectors

Level 1 vectors allow the assembly of up to five Level 0 assembly units into one Level 1 module. This is achieved by digestion of the Level 0 assembly units with one of the provided eight-cutters, clean-up of the DNA fragments and further combination of Level 0 fragments with appropriate, linearized Level 1 vector in an *in vitro* or *in vivo* assembly reaction. For this, Level 1 vectors with five different pairs of homology regions corresponding to the five Level 0 entry sets have been designed. In contrast to the Level 0 vectors, the Level 1 destination vectors confer ampicillin resistance, allowing efficient counter selection against undigested Level 0 backbones. Each Level 1 vector backbone is available in two versions, with either a high copy 2-micron origin or a low copy CEN/ARS region for replication in yeast. This boosts the flexibility for the direct expression of small assembly projects consisting of up to five units in a single Level 1 module. To create Level 1 vectors compatible with the different Level 0 vector sets, a homology containing region, flanked by two I-*Sce*I sites was inserted. This region consists of a 50 bp homology region on the left side, a *Pac*I site for linearization, and a promoter less auxotrophic marker gene, which contains the second homology region defined as the first 17 codons (51 bp). The marker gene is preceded by a third 50 bp homology region downstream of the terminator. The two homology regions directly flanking the *Pac*I site provide overlaps to the outmost homology regions from one Level 0 vector set, e.g. to A0 and AR from vector set A, and B0 and BR from vector set B, while the third homology region downstream of the marker gene is used for later Level 2 assembly.

The Level 1 assembly is done by *in vitro* methods or by *in vivo* assembly in yeast. The highly efficient, but more time consuming assembly in yeast is assisted by the use of promoterless markers on the Level 1 backbones, which become functional only upon recombination with the appropriate Level 0 assembly unit, thereby indicating a successful assembly. The homing endonuclease I-*Sce*I is used to release successfully assembled Level 1 modules from the vector backbone, without the need for PCR amplification.

### Design of Level 2 vectors

Up to five Level 1 modules, each assembled from up to five Level 0 units, are combined into a single Level 2 destination vector. These vectors are used directly to maintain and express the assembled units in *S. cerevisiae* either in high or low copy number.
The basic Level 2 vector backbones are identical to the Level 1 backbones and differ only in the homology regions inserted between the two I-*Sce*I sites.

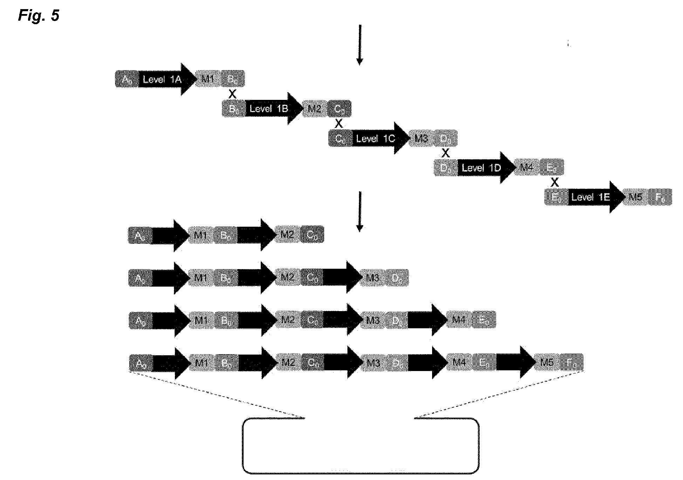

All Level 2 vectors contain homology region A0 in combination with the second region, providing overlap to the outmost homology region from the last Level 1 module that is part of the assembly, as shown in Fig. 5. For the final assembly step, Level 1 modules are released from the Level 1 backbone by digestion with I-*Sce*I*.* The released Level 1 modules contain all necessary homologies to each other and to the linearized Level 2 vector to allow a directed final assembly by TAR cloning. The successful assembly is monitored by selection of transformed yeast on SD dropout medium selecting for the presence of all incorporated Level 1 modules.

## Claims

1. Method for generating a multiple-gene assembly in a single DNA construct, which is **characterized by** the following steps:
a) Predetermining the order and orientation of subunits, which are provided as single- or double-stranded DNA, in the multiple-gene assembly in the single DNA construct to be generated,
b) Providing at least one Level 0 vector set containing at least two Level 0 vectors, which differ in their homology regions, at least one subunit per Level 0 vector and at least one Level 1 vector;
c) Providing at least one Level 2 DNA vector if at least two Level 1 vectors are provided in step b),
d) Adding overlaps to subunits in order to achieve the predetermined order and orientation of subunits,
e) Assembling the subunits of step d) into individual Level 0 vectors, depending on the predetermination in step a), chosen from one Level 0 vector set, by releasing a cassette, which separates the homology regions in each Level 0 vector, and cloning the predetermined subunit in-between the homology regions of each Level 0 vector by overlap based cloning,
f) Repeating steps d) and e) depending on the number of subunits provided in step b), whereas those steps are performed in parallel,
g) Releasing all Level 0 assembly units, which are the assembled subunits of all steps e) combined with the homology regions of the Level 0 vector, from each Level 0 vector backbone and isolating the released Level 0 assembly units,
h) Assembling the released Level 0 assembly units of step g) into one corresponding Level 1 vector by overlap based cloning between the released Level 0 assembly units and the corresponding Level 1 vector,
i) Repeating steps d) to h) in parallel for each Level 0 vector set provided in step b),
j) Releasing all Level 1 modules, which are the subcloned Level 0 assembly units combined with the homology regions of the corresponding Level 1 vector generated in steps h) and i) from the Level 1 vector backbone and isolating the released Level 1 modules,
k) Assembling of all released Level 1 modules of step j) into one single Level 2 DNA vector by overlap based cloning between the Level 1 modules and the Level 2 vector by recombination.

2. Method, according to claim 1, **characterized in that** a set of optimized 15-80 bp long overlap regions, whereby 15-36 bp long overlap regions are preferred, are added to specific subunits prior to step d), thereby allowing either predetermined or combinatorial assemblies with different subunits being randomly assembled at one or more positions of a Level 0 assembly unit.

3. Method, according to claim 1, **characterized in that** the vector backbone is identical for all Level 0 vectors in step e), whereas the homology regions vary in between the Level 0 vectors of one Level 0 vector set.

4. Method, according to claim 3, **characterized in that** the Level 0 vectors are positioning vectors, defined by their homology regions, whereby the choice of a Level 0 vector determines the position of the corresponding Level 0 assembly unit in the multiple-gene assembly to be generated.

5. Method, according to claims 3 or 4, **characterized in that** each Level 0 vector set contains one Level 0 vector with a first homology region, which is 15 bp to 80 bp long, whereby a length of 50 bp is preferred and corresponds to the left arm of a specific corresponding Level 1 vector and a second homology region, which is 15 bp to 80 bp long, whereby a length of 36 bp is preferred, and corresponds to the next Level 0 vector within the Level 0 vector set, whereas the homology regions of the other Level 0 vectors of one Level 0 vector set have inlying homology regions, which are both 15 bp to 80 bp long overlaps, whereby a length of 36 bp is preferred, and are required for the directed assembly of the Level 0 assembly units into single Level 1 vectors and whereas each Level 0 vector set contains one Level 0 vector with a last one homology region, which is a group of 15 bp to 80 bp sequences, whereby 51 bp sequences are preferred, and correspond to the right arm of the corresponding Level 1 vector, whereby this group of sequences each represents the first codons from different selectable or screenable marker genes, is preceded by the corresponding promoter region and followed by a stop codon.

6. Method, according to claim 1, **characterized in that** in step e) a Level 0 vector is used, which is universal, whereby the vector is defined by two non-variable homology regions, in-between those regions any subunits are assembled and the complete Level 0 assembly unit is afterwards released and assembled into a Level 1 vector at any desired position.

7. Method, according to claim 1, **characterized in that**, that in step h) the Level 1 vector contains one homology region, which is a 15 bp to 80 bp long region, whereby a 50 bp long region is preferred and a second homology region that is a group of 15 bp to 80 bp sequences, whereby 51 bp sequences are preferred, which correspond to the first codons of a selectable or screenable marker gene, in order to be compatible with the different Level 0 assembly units.

8. Method, according to claim 1, **characterized in that**, that in step k) the Level 2 vector contains a left homology region, which corresponds to the first homology region of the first Level 1 module and a second homology region, which corresponds to the last homology region of the last Level 1 module which is part of the gene assembly.

9. Method, according to claim 1, **characterized in that** the marker gene is different between the Level 0 vector and Level 1 vector and/or between the Level 1 vector and the Level 2 vector.

10. Method, according to claim1, **characterized in that** in a further step I) the Level 2 module, which are the combined Level 1 modules and homology regions of the Level 2 vector, is released from the Level 2 vector backbone and subcloned into varying vectors by either ligation based cloning or by overlap based cloning using the homology regions flanking the Level 2 module.

11. Method, according to claim 1, **characterized in that** any unit or module in Level 1 or Level 2 vectors is genome integrated by using a suitable nuclease along with donor DNA, if required.

12. Single DNA construct generated by the method according to any of the claims 1 to 11, **characterized in that** it contains freely designed scar-free Level 0 assembly units in a predefined order, determined by flanking homology regions and is sequence-independent.

13. Kit for generating a single DNA construct by using the method according to any of the claims 1 to 11 comprising at least one Level 0 vector set, containing at least two Level 0 vectors, which differ in their homology regions, at least one subunit per Level 0 vector, at least one Level 1 vector and a Level 2 vector.

14. Single DNA construct obtainable by
a) Predetermining the order and orientation of subunits, which are provided as single- or double-stranded DNA, in the multiple-gene assembly in the single DNA construct to be generated,
b) Providing at least one Level 0 vector set containing at least two Level 0 vectors, which differ in their homology regions, at least one subunit per Level 0 vector and at least one Level 1 vector;
c) Providing at least one Level 2 DNA vector if at least two Level 1 vectors are provided in step b),
d) Adding overlaps to subunits in order to achieve the predetermined order and orientation of subunits,
e) Assembling the subunits of step d) into individual Level 0 vectors, depending on the predetermination in step a), chosen from one Level 0 vector set, by releasing a cassette, which separates the homology regions in each Level 0 vector, and cloning the predetermined subunit in-between the homology regions of each Level 0 vector by overlap based cloning,
f) Repeating steps d) and e) depending on the number of subunits provided in step b), whereas those steps are performed in parallel,
g) Releasing all Level 0 assembly units, which are the assembled subunits of all steps e) combined with the homology regions of the Level 0 vector, from each Level 0 vector backbone and isolating the released Level 0 assembly units,
h) Assembling the released Level 0 assembly units of step g) into one corresponding Level 1 vector by overlap based cloning between the released Level 0 assembly units and the corresponding Level 1 vector,
i) Repeating steps d) to h) in parallel for each Level 0 vector set provided in step b),
j) Releasing all Level 1 modules, which are the subcloned Level 0 assembly units combined with the homology regions of the corresponding Level 1 vector generated in steps h) and i) from the Level 1 vector backbone and isolating the released Level 1 modules,
k) Assembling of all released Level 1 modules of step j) into one single Level 2 DNA vector by overlap based cloning between the Level 1 modules and the Level 2 vector by recombination.
